# EUROPEAN PATENT APPLICATION

(11) **EP 4 813 395 A1**
(43) Date of publication of application: **30.09.2026**
(21) Application number: 24894208.8
(22) Date of filing: 21.11.2024
(51) Int. Cl.: A61K 31/688, A61K 31/7125, A61K 48/00, A61P 13/12, C12N 15/113

(54) **PROPHYLACTIC AND/OR THERAPEUTIC AGENT FOR CYSTIC KIDNEY DISEASES**

(30) Priority: 22.11.2023 JP 2023197995
(71) Applicant: NATIONAL UNIVERSITY CORPORATION TOKAI NATIONAL HIGHER EDUCATION AND RESEARCH SYSTEM, Nagoya-shi, Aichi 464-8601 (JP)
(72) Inventor: KATO, Noritoshi, Nagoya-shi, Aichi 464-8601 (JP); ASANUMA, Hiroyuki, Nagoya-shi, Aichi 464-8601 (JP); MARUYAMA, Shoichi, Nagoya-shi, Aichi 464-8601 (JP); KATO, Yukiko, Nagoya-shi, Aichi 464-8601 (JP); NODA, Yuhei, Nagoya-shi, Aichi 464-8601 (JP); SATO, Fuminori, Nagoya-shi, Aichi 464-8601 (JP)
(74) Representative: Hoffmann Eitle
(86) International application number: PCT/JP2024/041339
(87) International publication number: WO 2025/110217

(57) **Abstract**

Provided is a prophylactic and/or therapeutic agent for cystic kidney disease.

The prophylactic and/or therapeutic agent for cystic kidney disease comprises a single-stranded oligonucleotide comprising a base sequence complementary to at least a portion of a base sequence of miR-21.

## Description

### Technical Field

The present disclosure relates to a prophylactic and/or therapeutic agent for cystic kidney disease, and the like. More specifically, the present disclosure relates to a prophylactic and/or therapeutic agent for cystic kidney disease, comprising a single-stranded oligonucleotide comprising a base sequence complementary to at least a portion of a base sequence of miR-21, and the like.

### Background Art

Autosomal dominant polycystic kidney disease, which is a type of cystic kidney disease, is a congenital disease caused by a single gene PKD1 or PDK2 mutation. The incidence is said to be 1 in 3000 people, and it is one of the most common single gene mutation diseases. Cysts form in the liver and kidneys, resulting in kidney enlargement, and approximately half of patients in their late 60s require maintenance dialysis.

miR-21 is an miRNA involved in cell proliferation, apoptosis suppression, enhancement of fibrosis, etc., and antisense oligonucleotides (anti-miRNA Oligo: AMO) targeting miR-21 for therapeutic purposes are being researched and developed.

It has also been reported that in an orthologous mouse model of ADPKD, knockout of miR-21 suppressed cyst growth (Non-patent Literature 1).

### Citation List

### Patent Literature

PTL 1: WO2021/153762
PTL 2: WO2023/140040

### Non-patent Literature

NPL 1: J Am Soc Nephrol. 2016 Aug; 27(8): 2319-30. doi: 10.1681/ASN.2015060634. Epub 2015 Dec 17.

### Summary of Invention

### Technical Problem

An object of the present disclosure is to provide a prophylactic and/or therapeutic agent for cystic kidney disease.

### Solution to Problem

The present inventors found that miR-21 expression is increased in the kidney tissue of a cystic disease model mouse and that cyst growth is suppressed by an miR-21 antisense oligonucleotide. The inventors further made improvements.

The present disclosure includes, for example, the subject matter described in the following items.

### Item 1.

A prophylactic and/or therapeutic agent for cystic kidney disease, comprising a single-stranded oligonucleotide comprising a base sequence complementary to at least a portion of a base sequence of miR-21.

### Item 1A.

A method for the prophylaxis and/or treatment of cystic kidney disease, comprising administering a single-stranded oligonucleotide comprising a base sequence complementary to at least a portion of a base sequence of miR-21 to a subject in need thereof (preferably a subject having cystic kidney disease).

### Item 1B.

A single-stranded oligonucleotide comprising a base sequence complementary to at least a portion of a base sequence of miR-21, for use in the prophylaxis and/or treatment of cystic kidney disease.

### Item 1C.

Use of a single-stranded oligonucleotide comprising a base sequence complementary to at least a portion of a base sequence of miR-21, for the production of a prophylactic and/or therapeutic agent for cystic kidney disease.

### Item 1D.

Use of a single-stranded oligonucleotide comprising a base sequence complementary to at least a portion of a base sequence of miR-21, for the prophylaxis and/or treatment of cystic kidney disease.

### Item 2.

The agent according to Item 1, wherein the single-stranded oligonucleotide comprises a modified nucleoside structural unit and/or a modified internucleoside linkage.

### Item 3.

The agent according to Item 2, wherein the modified nucleoside structural unit is an acyclic nucleoside structural unit and/or a locked nucleic acid (LAN) structural unit, or
the modified internucleoside linkage is a phosphorothioate linkage.

### Item 4.

The agent according to Item 3, wherein the acyclic nucleoside structural unit is a structural unit represented by the following formula (1): 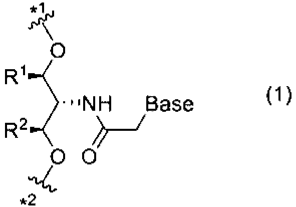 wherein R¹ and R² are the same or different, and each represents a hydrogen atom or an organic group, excluding a case in which R¹ and R² are both organic groups, and Base represents a nucleobase.

### Item 5.

The agent according to Item 4, wherein in formula (1), R¹ is a hydrogen atom or a methyl group, and R² is a hydrogen atom.

### Item 6.

The agent according to any one of Item 1 to 5, wherein in the single-stranded oligonucleotide,
a base complementary to at least one uracil selected from the group consisting of positions 5, 6, 8, 14, 17, 19, and 20 of the base sequence set forth in SEQ ID NO: 1 is 2,6-diaminopurine, and/or
a base complementary to at least one adenine selected from the group consisting of positions 7, 10, and 16 of the base sequence set forth in SEQ ID NO: 1 is 2-thiouracil.

### Item 7.

The agent according to Item 3, wherein
(1) the single-stranded oligonucleotide comprises a base sequence complementary to at least 16 bases, preferably at least 18 bases, of the base sequence of miR-21,
(2) a nucleoside structural unit forming the single-stranded oligonucleotide is a modified nucleoside structural unit represented by the following formula (1): 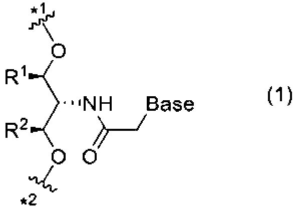 wherein R¹ and R² are the same or different, and each represents a hydrogen atom or an organic group, excluding a case in which R¹ and R² are both organic groups, and Base represents a nucleobase,
(3) in the single-stranded oligonucleotide, a base complementary to at least one uracil selected from the group consisting of positions 5, 6, 8, 14, 17, 19, and 20 of the base sequence set forth in SEQ ID NO: 1 is 2,6-diaminopurine,
(4) a base complementary to at least one adenine selected from the group consisting of positions 7, 10, and 16 of the base sequence set forth in SEQ ID NO: 1 is 2-thiouracil, and
(5) the number of phosphorothioate linkages is 20% or more based on the number of internucleoside linkages in the single-stranded oligonucleotide taken as 100%.

### Item 8.

The agent according to Item 7, wherein the single-stranded oligonucleotide is
(iii-a): an oligonucleotide in which all nucleoside structural units are SNA structural units and/or L-aTNA structural units, and the base sequence of the oligonucleotide consists of the base sequence set forth in SEQ ID NO: 6, or
(iii-b): an oligonucleotide comprising a base sequence in which one or more bases are deleted, substituted, or added in the base sequence of the oligonucleotide (iii-a).

### Item 9.

The agent according to any one of Items 1 to 6, wherein the single-stranded oligonucleotide is
(i-a): an oligonucleotide consisting of the base sequence set forth in SEQ ID NO: 2,
(i-b): an oligonucleotide comprising a base sequence in which one or more bases are deleted, substituted, or added in the base sequence of the oligonucleotide consisting of the base sequence set forth in SEQ ID NO: 2,
(ii-a): an oligonucleotide in which the base sequence of the oligonucleotide is GATAAG^{m}CT, wherein ^{m}C represents 5-methylcytosine, all nucleoside structural units are LNA structural units, and all internucleoside linkages are phosphorothioate linkages, or
(ii-b): an oligonucleotide comprising a base sequence in which one or more bases are deleted, substituted, or added in the base sequence of the oligonucleotide (ii-a).

### Advantageous Effects of Invention

Provided is a prophylactic and/or therapeutic agent for cystic kidney disease.

### Brief Description of Drawings

Fig. 1 shows the results of measuring miR-21 expression levels in mouse kidney tissue (n = 3 in each group; *P < 0.05; t-test).
Fig. 2 shows the results of miR-21 detection in mouse kidney tissue.
Fig. 3 shows the results of miR-21 detection in human kidney tissue.
Fig. 4 shows the results of evaluating pharmacokinetics of disulfoCy5-anti-miR-21 (SNA).
Fig. 5 shows the staining results of mouse kidney tissue.
Fig. 6 shows the results of measuring kidney weight/body weight (KW/BW) and blood urea nitrogen (BUN) concentration in mice.
Fig. 7 shows the results of measuring blood aspartate aminotransferase (AST) and alanine aminotransferase (ALT) in mice.
Fig. 8 shows the results of miR-21 detection in mouse kidney tissue after oligonucleotide administration (n = 5; *P < 0.05; one way-ANOVA).
Fig. 9 shows the results of measuring expression of PPARα and BCL2 in mouse kidney tissue.
Fig. 10 shows the results of measuring expression of TGF-β, SMAD7, and αSMA in mouse kidney tissue.
Fig. 11 shows the results of measuring the cyst size when primary culture of renal tubular cells was performed from kidneys removed from patients with human cystic kidney disease and three-dimensional culture was carried out.
Fig. 12 shows the results of measuring creatinine (Cre) concentration in mouse serum.
Fig. 13 shows the results of measuring kidney weight/body weight (KW/BW) and serum creatinine (Cre) concentration in mice.

### Description of Embodiments

Embodiments encompassed by the present disclosure are described in more detail below.

The prophylactic and/or therapeutic agent for cystic kidney disease encompassed by the present disclosure comprises a single-stranded oligonucleotide comprising a base sequence complementary to at least a portion of a base sequence of miR-21. In the present specification, the agent may be referred to as "the agent of the present disclosure" or the like. In addition, the single-stranded oligonucleotide may be referred to as "the single-stranded oligonucleotide of the present disclosure" or the like.

The base sequence set forth in SEQ ID NO: 1 is the base sequence (22 bases in length) that constitutes of miR-21.

The phrase "at least a portion of a base sequence of miR-21" is not particularly limited as long as the single-stranded oligonucleotide of the present disclosure can form a complementary strand with miR-21, and is, for example, 6 to 22 bases in length. The lower limit of the range is not particularly limited, and is preferably 7 bases in length, more preferably 8 bases in length, even more preferably 10 bases in length, still even more preferably 14 bases in length, particularly preferably 18 bases in length, and further particularly preferably 21 bases in length.

In the present specification, the term "complementary" includes not only complete base complementarity (completely complementary: hybridizing without mismatches, such as A and T or U, and G and C), but also complementarity to the extent that they can hybridize under stringent conditions. The stringent conditions can be determined based on the melting temperature (Tm) of nucleic acids, as taught by Berger and Kimmel (1987, Guide to Molecular Cloning Techniques Methods in Enzymology, Vol. 152, Academic Press, San Diego, CA). Examples of washing conditions after hybridization generally include about "1 × SSC, 0.1% SDS, 37°C." It is preferable that the hybridized state is maintained even after washing under such conditions. Although it is not particularly limited, examples of the washing conditions include more stringent hybridization conditions of about "0.5 × SSC, 0.1% SDS, 42°C," and even more stringent hybridization conditions of about "0.1 × SSC, 0.1% SDS, 65°C."

As stated above, since the complementarity is based on a one-to-one relationship between bases, a base sequence X and a base sequence Y complementary to the base sequence X have the same length of bases.

The number of bases constituting the single-stranded oligonucleotide of the present disclosure is preferably, for example, about 6 to 25. The lower limit of the range is not particularly limited, and is preferably 7, more preferably 8, even more preferably 10, still even more preferably 14, and particularly preferably 18. The upper limit of the range is not particularly limited, and is preferably 24, more preferably 23, even more preferably 22, and particularly preferably 21.

The single-stranded oligonucleotide of the present disclosure is an oligonucleotide comprising a base sequence having an identity of, for example, 85% or more, preferably 90% or more, more preferably 95% or more, even more preferably 98% or more, still even more preferably 99% or more, and particularly preferably 100% to the base sequence completely complementary to at least a portion of the base sequence of miR-21.

In the present specification, the term "identity" of base sequences refers to the degree to which two or more contrastable base sequences match each other. Thus, the higher the degree of match between two base sequences, the higher the identity or similarity of those sequences. The level of base sequence identity is determined, for example, by using FASTA, which is a tool for sequence analysis, with default parameters. Alternatively, the level of base sequence identity can be determined by using the BLAST algorithm by Karlin and Altschul (Karlin S., Altschul S. F. "Methods for assessing the statistical significance of molecular sequence features by using general scoring schemes," Proc. Natl. Acad. Sci. USA. 87: 2264-2268 (1990); Karlin S., Altschul S. F. "Applications and statistics for multiple high-scoring segments in molecular sequences." Proc. Natl. Acad. Sci. USA. 90: 5873-7 (1993)). A program called "BLASTX" based on this BLAST algorithm has been developed. The specific techniques of these analysis methods are known and can be found on the website of the National Center of Biotechnology Information (NCBI) (http://www.ncbi.nlm.nih.gov/).

The single-stranded oligonucleotide of the present disclosure is preferably an oligonucleotide that does not form a self-duplex. Examples of self-duplexes include intramolecular (hairpin) duplexes, intermolecular (dimer) duplexes, and the like. Oligonucleotides that do not form self-duplexes can be obtained, for example, by not including a base sequence portion capable of forming a self-duplex, by introducing a substitution and/or any change or the like into a base sequence portion capable of forming a self-duplex, or the like.

The single-stranded oligonucleotide of the present disclosure may be DNA, RNA, or the like, and can comprise a modified nucleoside structural unit and/or a modified internucleoside linkage based thereon.

The modified nucleoside structural unit is not particularly limited as long as it is a structural unit that corresponds to a nucleoside constituting the oligonucleotide. Examples of modified nucleoside structural units include molecules obtained by chemically modifying ribonucleosides, deoxyribonucleosides, DNA, RNA, etc. Examples include nucleosides modified with 2'-OMe, nucleosides modified with 2'-MOE, and sugar-modified nucleoside structural units, such as LNA (registered trademark), ENA (registered trademark), AmNA (registered trademark), GuNA, and scpBNA.

A "modified sugar" refers to a sugar having any substitution and/or any change compared to a natural sugar moiety (i.e., a sugar moiety found in DNA (2'-H) or RNA (2'-OH)). A "sugar-modified nucleoside" refers to a modified nucleoside containing a modified sugar. The sugar-modified nucleoside may be any nucleoside in which all or part of the chemical structure of the sugar moiety is modified by addition or substitution of any chemical moiety. Examples include a modified nucleoside substituted with 2'-O-methyl in the sugar moiety, a modified nucleoside substituted with 2'-O-propyl in the sugar moiety, a modified nucleoside substituted with 2'-methoxyethoxy in the sugar moiety, a modified nucleoside substituted with 2'-O-methoxyethyl in the sugar moiety, a modified nucleoside substituted with 2'-O-[2-(guanidinium)ethyl] in the sugar moiety, and a modified nucleoside substituted with 2'-fluoro in the sugar moiety, morpholino nucleic acids (morpholinos, commonly known as PMO), which are modified nucleosides in which the sugar moiety is substituted with a morpholine ring, and bridged nucleic acids (BNA) having two cyclic structures obtained by introducing a bridged structure into the sugar moiety, such as a locked nucleic acid (LNA), in which the oxygen atom at the 2'-position and the carbon atom at the 4'-position are bridged via methylene, and an ethylene bridged nucleic acid (ENA) (Nucleic Acids Research, 32, e175 (2004)). Examples further include a peptide nucleic acid (PNA) (Acc. Chem. Res., 32, 624 (1999)), an oxypeptide nucleic acid (OPNA) (J. Am. Chem. Soc., 123, 4653 (2001)), and a peptide ribonucleic acid (PRNA) (J. Am. Chem. Soc., 122, 6900 (2000)). Furthermore, as described below, acyclic nucleoside structural units can also be used as modified nucleoside structural units.

Other modified nucleosides that may be used include molecules in which an atom (e.g., a hydrogen atom, an oxygen atom) or a functional group (e.g., a hydroxyl group, an amino group) of a base moiety of a nucleic acid is substituted with another atom (e.g., a hydrogen atom, a sulfur atom), a functional group (e.g., an amino group), or an alkyl group having 1 to 6 carbon atoms, or protected with a protecting group (e.g., a methyl group or an acyl group); molecules in which another chemical substance, such as a lipid, a phospholipid, phenazine, folate, phenanthridine, anthraquinone, acridine, fluorescein, rhodamine, coumarin, or a dye, is added to a nucleoside; and the like.

Preferred modified nucleoside structural units are LNA structural units and/or acyclic nucleoside structural units, and more preferred modified nucleoside structural units are acyclic nucleoside structural units.

The modified nucleoside structural units may be used singly or in a combination of two or more.

The proportion of the modified nucleoside structural unit(s) is for example, 50% or more, preferably 70% or more, more preferably 80% or more, even more preferably 90% or more, still even more preferably 95%, and particularly preferably 100%, based on the nucleoside structural units (the number of nucleosides) constituting the single-stranded oligonucleotide of the present disclosure taken as 100%.

In one embodiment, the single-stranded oligonucleotide of the present disclosure preferably comprises one or more acyclic nucleoside structural units. The proportion of the acyclic nucleoside structural unit(s) is, for example, 50% or more, preferably 70% or more, more preferably 80% or more, even more preferably 90% or more, still even more preferably 95%, and particularly preferably 100%, based on the nucleoside structural units (the number of nucleosides) constituting the single-stranded oligonucleotide of the present disclosure taken as 100%.

In the present specification, "acyclic nucleoside structural unit" is a structural unit that corresponds to a nucleoside constituting the oligonucleotide, and is not particularly limited as long as it does not contain a sugar skeleton and has an acyclic skeleton. Typical examples of acyclic nucleoside structural units include a structural unit represented by formula (1): 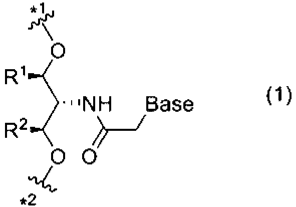 wherein R¹ and R² are the same or different, and each represents a hydrogen atom or an organic group, excluding a case in which R¹ and R² are both organic groups, and Base represents a nucleobase.

The organic group is not particularly limited, and examples include hydrocarbon groups.

Preferred examples of hydrocarbon groups include chain hydrocarbon groups. Examples of chain hydrocarbon groups include alkyl, alkenyl, and alkynyl groups. Of these, for example, alkyl groups are preferable. Specific examples of alkyl groups include methyl, ethyl, n-propyl, isopropyl, n-butyl, isobutyl, tert-butyl, sec-butyl, n-pentyl, neopentyl, n-hexyl, and 3-methylpentyl groups. Of these, methyl groups are preferable. The number of carbon atoms in the hydrocarbon group is not particularly limited. The number of carbon atoms is preferably 1 to 8, more preferably 1 to 6, even more preferably 1 to 4, still even more preferably 1 or 2, and particularly preferably 1. Further preferred is an alkyl group containing an alkynyl group (-C≡C-, -C=CH) at the end or inside, which enables the introduction of various functional groups by click reactions etc.

In addition to the above examples, the organic group may be a monovalent group obtained by removing one hydrogen atom or functional group from various molecules, such as molecules used to modify nucleosides. Examples of such molecules include polyethylene glycol chains, dye molecules, polycation (spermine), groove binders, amino groups, hydroxyl groups, thiol groups, metal ligands, photocleavable functional groups, sugar chains, and the like. These can be linked directly or indirectly to the skeleton of the above structural unit. For example, click reactions (e.g., the reaction of alkyne and azide described above) can be used for linkage.

In a preferred embodiment of the present disclosure, in terms of binding properties to miR-21, it is preferable that R¹ is a hydrogen atom or a chain hydrocarbon group (preferably a methyl group) and that R² is a hydrogen atom. In other words, preferred examples of acyclic nucleoside structural units include SNA (serinol nucleic acid) structural units (R¹ and R² are hydrogen atoms) and/or L-aTNA (acyclic L-threoninol nucleic acid) structural units (R¹ is a methyl group, and R² is a hydrogen atom) .

As the nucleobase, bases constituting nucleic acids can be used without restriction. Bases constituting nucleic acids include not only typical bases found in RNA, DNA, and other naturally occurring nucleic acids (e.g., adenine (A), thymine (T), uracil (U), guanine (G), and cytosine (C)), but also other bases, such as hypoxanthine (I) and modified bases. Examples of modified bases include 2,6-diaminopurine, 2-thiouracil, 2-thiothymine, pseudouracil, 3-methyluracil, dihydrouracil, 5-alkylcytosine (e.g., 5-methylcytosine), 5-alkyluracil (e.g., 5-ethyluracil), 5-halouracil (e.g., 5-bromouracil), 6-azapyrimidine, 6-alkylpyrimidine (e.g., 6-methyluracil), 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methoxyuracil, 5-methyl-2-thiouracil, 2-methylthio-N6-isopentenyladenine, uracil-5-oxyacetic acid, 2-thiocytosine, purine, 2-aminopurine, isoguanine, indole, imidazole, xanthine, and the like.

In formula (1), *1 and *2 indicate the directions in which an oligonucleotide is constituted. When R¹ is an organic group and R² is a hydrogen atom, *1 is the 3'-side and *2 is the 1'-side. When R¹ is a hydrogen atom and R² is an organic group, *1 is the 1'-side and *2 is 3'-side. When R¹ is a hydrogen atom and R² is a hydrogen atom, *1 is the (S)-side and *2 is the (R)-side.

The (S)-end and (R)-end (SNA in formula (1) in paragraph [0046]) and the above 3'-side and 1'-side correspond to the 5' end and 3' end of DNA (LNA in the formula in paragraph [0071]), respectively. For convenience, the (S)-end and (R)-end and the 3'-side and 1'-side may be referred to as "the 5' end and 3' end," respectively.

When the modified nucleoside structural unit represented by formula (1) is located at a terminus, a hydrogen atom, a sulfur atom, or a phosphate group is added to its *1 and *2, according to the above.

The term "modified internucleoside linkage" refers to an internucleoside linkage having any substitution or any change compared to a naturally occurring internucleoside linkage (i.e., a phosphodiester linkage). The modified internucleoside linkages include internucleoside linkages containing a phosphorus atom and internucleoside linkages not containing a phosphorus atom. The modified internucleoside linkage may also be one in which any chemical substance is added or substituted on part or all of the chemical structure of the nucleotide phosphodiester bond. Examples of modified internucleoside linkages include a phosphorothioate linkage (also referred to as a "thiophosphate linkage"), a phosphorodithioate linkage, a phosphotriester linkage, a methylphosphonate linkage, a methylthiophosphonate linkage, a boranophosphate linkage, a phosphoramidate linkage, and the like. This is expected to prevent degradation by hydrolytic enzymes, such as nucleases.

The modified internucleoside linkage is particularly preferably a phosphorothioate linkage.

The modified internucleoside linkages may be used singly or in a combination of two or more.

The number of modified internucleoside linkages based on the number of internucleoside linkages in the single-stranded oligonucleotide of the present disclosure taken as 100% is not particularly limited, and is, for example, 20% or more, preferably 50% or more, more preferably 60% or more, even more preferably 70% or more, still more preferably 80% or more, and particularly preferably 90% or more. In one embodiment, all internucleoside linkages may be modified internucleoside linkages (e.g., phosphorothioate linkages).

In one embodiment, the single-stranded oligonucleotide of the present disclosure may be a single-stranded oligonucleotide in which a base complementary to at least one uracil in the base sequence set forth in SEQ ID NO: 1 is 2,6-diaminopurine, and/or a base complementary to at least one adenine in the base sequence set forth in SEQ ID NO: 1 is 2-thiouracil. The complementarity is the relationship in which miR-21 and the single-stranded oligonucleotide of the present disclosure are positioned o pposite each other when arranged in a complementary manner. By positioning 2,6-diaminopurine and 2-thiouracil opposite each other, it is expected that the formation of a self-duplex in the single-stranded oligonucleotide is suppressed, while further enhancing the binding properties to miR-21.

Examples of uracils in the base sequence set forth in SEQ ID NO: 1 include the uracils at positions 1, 5, 6, 8, 14, 17, 19, and 20. In the single-stranded oligonucleotide of the present disclosure, it is preferable that a base complementary to at least one uracil selected from the group consisting of positions 5, 6, 8, 14, 17, 19, and 20 is 2,6-diaminopurine. For example, the bases complementary to the uracils at positions 5, 6, 8, 14, 17, 19, and 20 may be 2,6-diaminopurines. The proportion of 2,6-diaminopurine contained in the single-stranded oligonucleotide of the present disclosure is, for example, 40 to 100%, preferably 50 to 100%, and more preferably 60 to 100%, based on the number of uracils contained in at least a portion of the base sequence of miR-21 taken as 100%.

Examples of adenines in the base sequence set forth in SEQ ID NO: 1 include the adenines at positions 2, 7, 10, 12, 16, and 22. In the single-stranded oligonucleotide of the present disclosure, it is preferable that a base complementary to at least one adenine selected from the group consisting of positions 7, 10, and 16 is 2-thiouracil. For example, the bases complementary to the adenines at positions 7, 10, and 16 may be 2-thiouracils.

The proportion of 2-thiouracil contained in the single-stranded oligonucleotide of the present disclosure is, for example, 20 to 100%, preferably 30 to 100%, and more preferably 40 to 100%, based on the number of adenines contained in at least a portion of the base sequence of miR-21 taken as 100%.

In one embodiment, the single-stranded oligonucleotide of the present disclosure has preferably one or more pairs, more preferably two or more pairs, even more preferably three or more pairs, of 2,6-diaminopurine and 2-thiouracil that are opposite each other. The relationship in which 2,6-diaminopurine and 2-thiouracil are opposite each other may be a relationship within a single-stranded oligonucleotide of the present disclosure, or may be a relationship between single-stranded oligonucleotides of the present disclosure. In particular, the relationship in which 2,6-diaminopurine and 2-thiouracil are opposite each other is preferably a relationship within a single-stranded oligonucleotide of the present disclosure.

A preferred embodiment of the single-stranded oligonucleotide of the present disclosure is, for example, an oligonucleotide (SEQ ID NO: 6) having the following base sequence: (S) -C DDC DsUCDG T CsUGDsU DDG CTA- (R), wherein D represents 2,6-diaminopurine, and sU represents 2-thiouracil.

A preferred embodiment of the single-stranded oligonucleotide of the present disclosure is, for example, an oligonucleotide that satisfies at least one, two, three, four, or all (five) of the following (1) to (5):
(1) the single-stranded oligonucleotide comprises a base sequence complementary to at least 18 bases of the base sequence of miR-21;
(2) a nucleoside structural unit forming the single-stranded oligonucleotide is a modified nucleoside structural unit represented by the following formula (1): 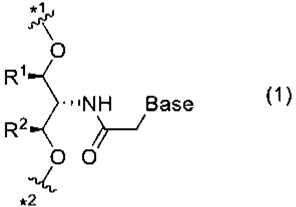 wherein R¹ and R² are the same or different, and each represents a hydrogen atom or an organic group, excluding a case in which R¹ and R² are both organic groups, and Base represents a nucleobase;
(3) in the single-stranded oligonucleotide, a base complementary to at least one uracil selected from the group consisting of positions 5, 6, 8, 14, 17, 19, and 20 of the base sequence set forth in SEQ ID NO: 1 is 2,6-diaminopurine;
(4) a base complementary to at least one adenine selected from the group consisting of positions 7, 10, and 16 of the base sequence set forth in SEQ ID NO: 1 is 2-thiouracil; and
(5) the number of phosphorothioate linkages is 20% or more based on the number of internucleoside linkages in the single-stranded oligonucleotide taken as 100%.

Preferred embodiments of the single-stranded oligonucleotide of the present disclosure include, for example, an oligonucleotide (SEQ ID NO: 2) in which the oligonucleotide has the following base sequence: (S) -C DDC DsUCDG T CsUGDsU DDG CTA- (R) (wherein D represents 2,6-diaminopurine, and sU represents 2-thiouracil), all nucleoside structural units are SNA structural units, and all internucleoside linkages are phosphorothioate linkages; an oligonucleotide (SEQ ID NO: 3) in which the oligonucleotide has the following base sequence: (S) -C DDC DsUCDG T CsUGDsU DDG CTA- (R) (wherein D represents 2,6-diaminopurine, and sU represents 2-thiouracil), all nucleoside structural units are L-aTNA structural units, and all internucleoside linkages are phosphorothioate linkages; an oligonucleotide (SEQ ID NO: 5) in which the oligonucleotide has the following base sequence: (S)-C* D*D*C* DsUCDG T*C*sU*GDsU DDG CT*A- (R) (SEQ ID NO: 5) (wherein D represents 2,6-diaminopurine, sU represents 2-thiouracil, and the symbol "*" between bases indicates that the linkage between nucleosides is a phosphorothioate linkage), and all nucleoside structural units are SNA structural units; and the like. In the present specification, these oligonucleotides maybe referred to as an "oligonucleotide consisting of the base sequence set forth in SEQ ID NO: 2," an "oligonucleotide consisting of the base sequence set forth in SEQ ID NO: 3," and the like.

In addition, a preferred embodiment of the single-stranded oligonucleotide of the present disclosure includes, for example, an oligonucleotide comprising a base sequence in which one or more bases are deleted, substituted, or added in the base sequence of the oligonucleotide set forth in SEQ ID NO: 2, 3, 5, or 6. The upper limit of the number of bases to be deleted, substituted, or added is not particularly limited as long as the single-stranded oligonucleotide of the present disclosure comprises a base sequence complementary to at least a portion of the base sequence of miR-21, and may be, for example, 10, 9, 8, 7, 6, 5, 4, or 3.

A preferred embodiment of the single-stranded oligonucleotide of the present disclosure includes, for example, an oligonucleotide having the following base sequence: 5' -GAT AAG^{m}CT- 3' (wherein ^{m}C represents 5-methylcytosine), all nucleoside structural units are LNA structural units, and all internucleoside linkages are phosphorothioate linkages.

A preferred embodiment of the single-stranded oligonucleotide of the present disclosure includes, for example, an oligonucleotide comprising a base sequence in which one or more bases are deleted, substituted, or added in the base sequence of an oligonucleotide in which the base sequence is 5' - GAT AAG^{m}CT- 3' (wherein ^{m}C represents 5-methylcytosine), all nucleoside structural units are LNA structural units, and all internucleoside linkages are phosphorothioate linkages. The upper limit of the number of bases to be deleted, substituted, or added is not particularly limited as long as the single-stranded oligonucleotide of the present disclosure comprises a base sequence complementary to at least a portion of the base sequence of miR-21, and may be, for example, 4 or 3.

The single-stranded oligonucleotide of the present disclosure may have another molecule linked thereto. Other molecules are not particularly limited, and examples include fluorescently labeled substances, biotin, azide, ethynyl groups, and the like. Examples of fluorescently labeled substances include fluorescein, rhodamine, Texas Red, tetramethylrhodamine, carboxyrhodamine, phycoerythrin, 6-FAM (Trademark), Cy (Registered trademark) 3, Cy (Registered trademark)5, Alexa Fluor (Registered trademark) series, and the like.

The single-stranded oligonucleotide of the present disclosure can be easily produced according to known genetic engineering techniques, artificial nucleic acid production techniques, and the like. For example, PCR, restriction enzyme cleavage, a nucleic acid ligation technique, and the like may be used for production.

The agent of the present disclosure preferably comprises the single-stranded oligonucleotide of the present disclosure as an active ingredient.

The content of the single-stranded oligonucleotide of the present disclosure in the agent of the present disclosure is not particularly limited, and may be, for example, about 0.0001 to 100 wt%.

The agent of the present disclosure comprises the single-stranded oligonucleotide of the present disclosure and may further comprise other components. Examples of the other components include pharmacologically acceptable bases, carriers, solvents, dispersants, emulsifiers, buffers, stabilizers, excipients, binders, disintegrants, lubricants, thickeners, moisturizers, colorants, fragrances, chelating agents, and the like.

The agent of the present disclosure can be used for the prophylaxis and/or treatment of cystic kidney disease.

Examples of cystic kidney disease include polycystic kidney disease and the like. Examples of polycystic kidney disease include autosomal dominant polycystic kidney disease (ADPKD), autosomal recessive polycystic kidney disease (ARPKD), and the like. Of these, ADPKD is preferable.

Examples of subjects to which the agent of the present disclosure is applied include humans and non-human mammals (such as rats, mice, rabbits, bovine, pigs, dogs, cats, sheep, and monkeys) .

The agent of the present disclosure can be in any dosage form, such as an oral dosage form, such as tablets (including orally disintegrating tablets, chewable tablets, effervescent tablets, troches, jelly drops, etc.), pills, granules, fine granules, powders, hard capsules, soft capsules, dry syrup, liquids (including drinks, suspensions, and syrup), and jellies; or a parenteral dosage form, such as injectable preparations (e.g., drip infusions (e.g., preparations for intravenous drip infusion), intravenous injections, intramuscular injections, subcutaneous injections, and intradermal injections), external preparations (e.g., ointments, cataplasms, and lotions), suppositories, inhalants, eye drops, ophthalmic ointments, nasal drops, and ear drops. The active ingredient can be administered in a state of being complexed with particles (e.g., lipid particles or exosomes), or in a state of being encapsulated in the particles.

The administration route for the agent of the present disclosure is not particularly limited as long as desired effects are achieved. Administration routes include oral administration, tube feeding, and enteral administration, such as enema administration; and parenteral administration, such as intravenous administration, intra-arterial administration, intramuscular administration, intracardiac administration, subcutaneous administration, intradermal administration, intraperitoneal administration, and intranasal administration.

The dosage of the agent of the present disclosure is not particularly limited as long as it is an amount effective to exhibit drug efficacy. Typically, the dosage for oral administration on an active-ingredient weight basis is 0.1 to 1000 mg/kg body weight per day, preferably 0.5 to 500 mg/kg body weight per day, whereas the dosage for parenteral administration on an active-ingredient weight basis is 0.01 to 100 mg/kg body weight per day, and preferably 0.05 to 50 mg/kg body weight per day. The dosage and administration interval above can be appropriately increased or decreased according to the age, pathological conditions, symptoms, and the like of the animal.

Without wishing to be bound by theory, it is believed that the single-stranded oligonucleotide of the present disclosure can prevent and/or treat cystic kidney disease by suppressing the enlargement of kidney cysts and/or liver cysts through, for example, promotion of PPARα expression, suppression of fibroblasts (e.g., promotion of SMAD7 expression, suppression of TGF-β expression, and/or suppression of αSMA expression, etc.), and/or suppression of expression of BCL2, which is an anti-apoptotic protein.

In the present specification, the term "comprising" includes not only "comprising" but also "consisting essentially of" and "consisting of." Furthermore, the present disclosure encompasses all possible combinations of the constituent elements described in the present specification.

Various characteristics (properties, structures, functions, etc.) described in the above embodiments of the present disclosure may be combined in any manner to specify the subject matter included in the present disclosure. That is, the present disclosure includes all of the subject matter comprising any combination of the combinable properties described herein.

### Examples

The present disclosure is described in detail below with reference to experimental examples; however, the present disclosure is not limited to these examples. In the following, experiments were performed at atmospheric pressure and ordinary temperature, unless otherwise specified. Unless otherwise specified, "%" indicates "mass%."

### Animal Model of Cystic Disease

Male DBA/2 mice (Japan SLC, Inc.), and male DBA/2FG-pcy mice (Kyudo Co., Ltd.) as cystic disease model mice, were used.

This study was approved by the Institutional Animal Care and Use Committee of the Nagoya University School of Medicine and was conducted in accordance with the animal experiment guidelines of the Nagoya University School of Medicine.

### Experimental Design

Male DBA/2 mice and male DBA/2FG-pcy mice were subcutaneously injected with a test agent at a dose of 10 mg/kg three times per week for 6 weeks from 4 weeks of age to 10 weeks of age. Under isoflurane anesthesia, the test agent was injected into the subcutaneous tissue below the left scapula from the dorsal side. After a total of 18 injections over 6 weeks, the mice were sacrificed, and kidney weight/body weight (KW/BW), blood tests, and kidney tissue were evaluated.

### Human Tissue

Normal human kidney tissue and kidney tissue from ADPKD patients, both removed at Nagoya University Hospital, were used as specimens. Consent for research use was obtained from the patients in accordance with the regulations at Nagoya University Hospital.

### qRT-PCR Analysis

Total RNA was extracted from tissue samples using TRIzol Reagent (Thermo Fisher Scientific). For miRNA detection, total RNA was reverse-transcribed using a TaqMan MicroRNA Reverse Transcription Kit (Thermo Fisher Scientific), and analysis was performed using TaqMan MicroRNA Assays (Thermo Fisher Scientific).

The target was amplified by real-time PCR using a Step One Plus Real-Time PCR System (Thermo Fisher Scientific) and Fast Advanced Master Mix (Thermo Fisher Scientific). All samples were measured in duplicate. Quantitative evaluation of target expression was performed using the ΔΔCT method, and miRNA expression levels were normalized relative to the expression level of U6 snRNA.

### miRNAscope Assay

To detect miR-21 in tissue, miRNAscope HD Reagent Kit - RED (Advanced Cell Diagnostics) was used. miRNAscope Positive Control Probe - SR-RNU6-S1 (Advanced Cell Diagnostics) was used as a positive control probe, and miRNAscope Negative Control Probe - SR-Scramble-S1 (Advanced Cell Diagnostics) was used as a negative control probe. miRNAscope Probe - SR-mmu-miR-21a-5q-S1 (Advanced Cell Diagnostics) was used to detect miR-21 in mouse kidney tissue. miRNAscope Probe - SR-hsa-miR-21-5p-S1 (Advanced Cell Diagnostics) was used to detect miR-21 in human kidney tissue. Tissue specimens stained with the kit were observed using BZ-X810 (Keyence).

### Anti-miR-21 (SNA), Anti-miR-21 (LNA), Scramble

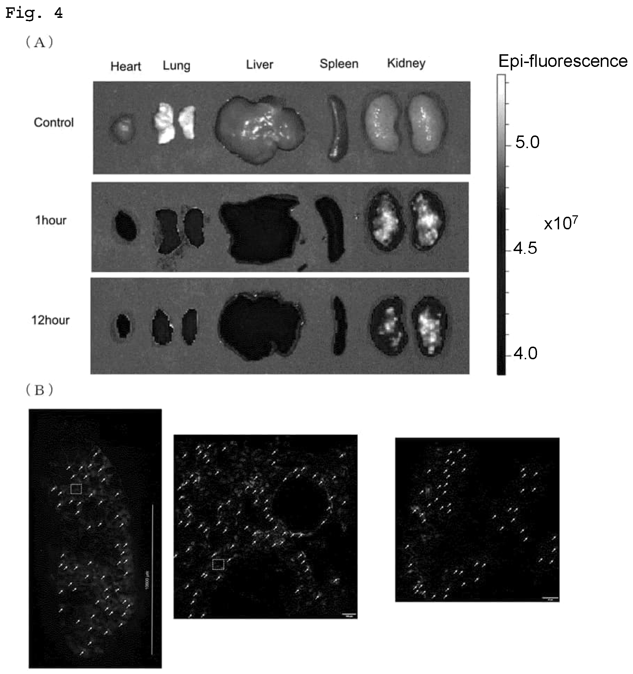

The antisense oligonucleotides of miR-21 (anti-miR-21 (SNA) and anti-miR-21(LNA): Figs. 4 to 11, scramble: Figs. 4 to 13) used as test agents in the tests shown in Figs. 4 to 13 described below are as follows.

The structural formula of SNA used is shown below.

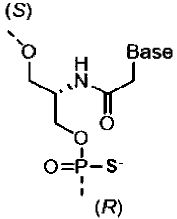

The base sequences of the anti-miR-21 (SNA; serinol nucleic acid) and scramble are as follows. In both sequences, all of the constituent nucleotides are SNAs.
Anti-miR-21 (SNA) (S-D7sU3): (S) -C DDC DsUCDG T CsUGDsU DDG CTA-(R) (SEQ ID NO: 2)
Scramble: (S) -TAT GAT GTC CAT GTC GTA CGC- (R) (SEQ ID NO: 4)

In the base sequence of the anti-miR-21 (SNA), D represents 2,6-diaminopurine, and sU represents 2-thiouracil. The anti-miR-21 (SNA) and scramble were synthesized by the method described in PTL 2 (a method using a protecting group removable by a base) and provided by the Asanuma Laboratory, School of Engineering, Nagoya University.

The structural formula of LNA used is shown below.

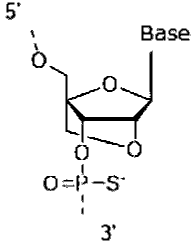

The base sequence of the anti-miR-21 (LNA; locked nucleic acid) is as follows. In this sequence, all of the constituent nucleotides are LNAs.
Anti-miR-21 (LNA) : 5' -GAT AAG^{m}CT- 3', wherein ^{m}C represents 5-methylcytosine.

The anti-miR-21 (LNA) was purchased from Gene Design and used.

In all of the anti-miR-21 (SNA), scramble, and anti-miR-21 (LNA), all of the internucleoside linkages were phosphorothioate (PS) linkages, as shown in the structural formulas.

### S-45371

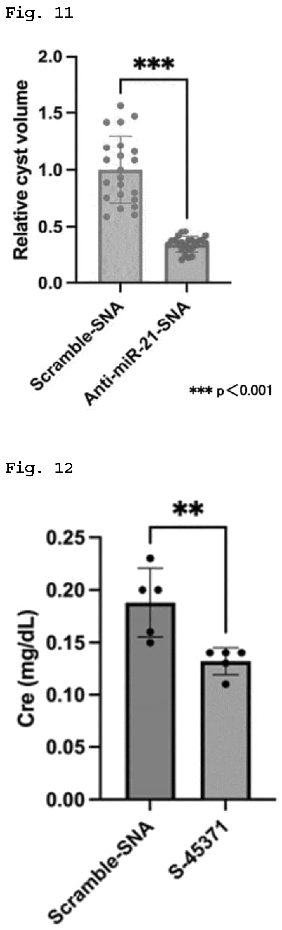

The miR-21 antisense oligonucleotide (S-45371) used as a test agent in the test shown in Fig. 12 below is as follows. In this oligonucleotide, all of the constituent nucleotides are SNAs.

### S-45371: (S)-C* D*D*C* DsUCDG T*C*sU*GDsU DDG CT*A-(R) (SEQ ID NO: 5)

In the base sequence of S-45371, D and sU are as defined above, each symbol "*" between bases indicates that the internucleoside linkage is a phosphorothioate linkage, and the absence of the symbol "*" between bases indicates that the internucleoside linkage is a phosphodiester linkage. The synthesis of S-45371 was performed in the same manner as for the anti-miR-21 (SNA) and scramble.

### T-D7sU3(PS)

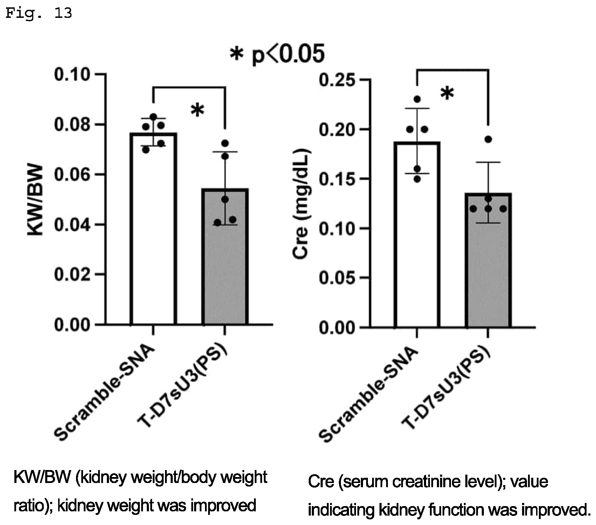

The miR-21 antisense oligonucleotide (T-D7sU3(PS)) used as a test agent in the test shown in Fig. 13 below is as follows. In this oligonucleotide, all of the constituent nucleotides are L-aTNAs.
T-D7sU3(PS): (S)-C DDC DsUCDG T CsUGDsU DDG CTA-(R) (SEQ ID NO: 3)

In the base sequence of T-D7sU3(PS), D and sU are as defined above. In addition, all of the internucleoside linkages are phosphorothioate linkages. The synthesis of T-D7sU3(PS) was performed in the same manner as for the anti-miR-21 (SNA) and scramble.

### Ex vivo Imaging

Anti-miR-21 (SNA) labeled with disulfoCy5 was used (provided by Asanuma Laboratory). Mice were sacrificed 1 hour and 12 hours after administration of the SNA. Fresh organs were collected and placed in an IVIS Spectrum in vivo imaging system (PerkinElmer, Waltham, MA, USA). Cy5 was excited at 643 nm and detected at 667 nm, and the data were measured as photon·sec·⁻¹cm⁻². Fluorescent tissue specimens were observed using BZ-X810 (Keyence) and SpinSR10 (Olympus Life Science).

### Histological Analysis

Organs were fixed in 10% paraformaldehyde, embedded in paraffin, cut into 4-µm-thick sections, and stained with hematoxylin-eosin.

### Blood Biochemical Analysis

The concentration of blood urea nitrogen (BUN) in serum and the serum creatinine (Cre), aspartate aminotransferase (AST), and alanine aminotransferase (ALT) concentrations were measured using an automated chemical analyzer (SRL, Inc., Tokyo, Japan).

### Western Blot Analysis

A β-actin antibody (4917; Cell Signaling), a PEARα antibody (PA1-822A; Invitrogen), a BCL2 antibody (ab182858; Abcam), a TGF-β antibody (3711; Cell Signaling), an SMAD7 antibody (42-0400; Invitrogen), and an αSMA antibody (19245; Cell Signaling) were used as primary antibodies.

### Three-Dimensional Culture of Renal Tubular Cells and Evaluation of Cyst Formation Suppression Effect of Antisense Introduction

Madin-Darby canine kidney (MDCK) cells were purchased from ATCC (CCL-34; American Type Culture Collection) and cultured at 37°C and 5% CO2 in Eagle's Minimum Essential Medium (051-07615; Fujifilm Wako) supplemented with 10% heat-inactivated fetal bovine serum. Primary cultured human ADPKD renal tubular cells were prepared from human ADPKD kidneys of inpatients at Nagoya University Hospital and cultured at 37°C and 5% CO2 in Dulbecco's modified Eagle medium/nutrient mixture F-12 (048-29785; Fujifilm Wako) supplemented with L-glutamine, 15 mM HEPES (D8437; Sigma-Aldrich), 1% penicillin-streptomycin (15140122; Thermo Fisher Scientific), and 1% insulin-transferrin-selenium (41400045; Thermo Fisher Scientific). The MDCK2 cells and primary cultured human ADPKD renal tubular cells were seeded in a 6-well plate at a density of 0.3 × 10⁶ cells/mL. After two days, when the cells reached 80% confluence, they were transfected with Scramble-SNA and Anti-miR-21-SNA using Lipofectamine 3000 Transfection Reagent (L3000008; Invitrogen). After 48 hours, the cells were collected and seeded onto Matrigel (356234; Corning) in a 24-well plate at a density of 1.0 × 10⁴ cells/mL, followed by treatment with 10 µM Forskolin (067-2191; Fujifilm Wako) for 48 hours. The cells were then observed using a BZ-X810 microscope (Keyence). Images were taken randomly, and the size and number of cysts were measured. Cysts and cell areas were analyzed using Image J (13.0.6).

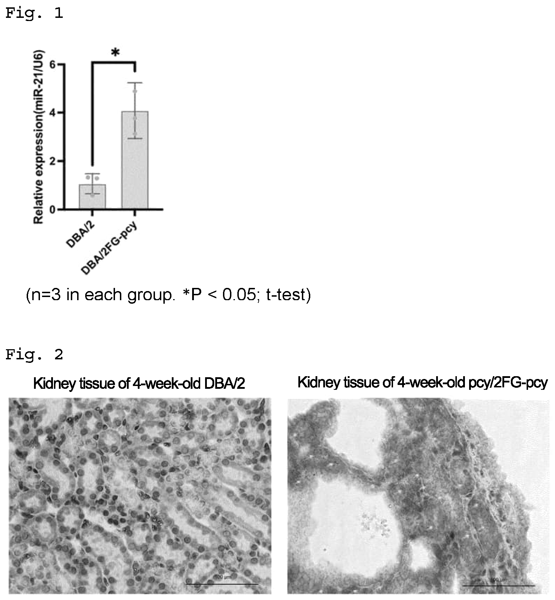

The expression of miR-21 in the kidney tissues of DBA/2 mice and DBA/2FG-pcy mice was analyzed by qRT-PCR. As shown in Fig. 1, it was confirmed that the expression of miR-21 was increased in the kidney tissue of the cystic kidney model mice.

The expression of miR-21 in the kidney tissues of DBA/2 mice and DBA/2FG-pcy mice was analyzed using miRNAscope. As shown in Fig. 2, it was confirmed that the expression of miR-21 was increased in the kidney tissue of the cystic disease model mice compared with the normal mouse kidney tissue.

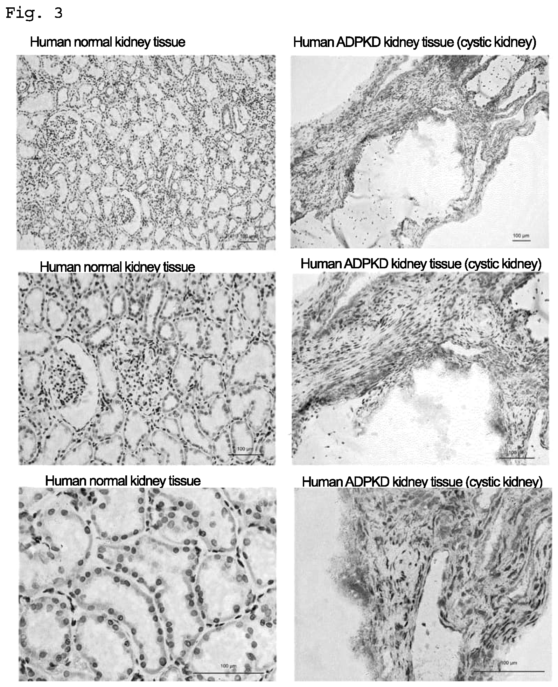

Furthermore, the expression of miR-21 in normal human kidney tissue and ADPKD patient kidney tissue was analyzed using miRNAscope. As shown in Fig. 3, it was confirmed that the expression of miR-21 was increased in the ADPKD patient kidney tissue compared with the normal human kidney tissue.

The above results confirmed that in cystic kidney disease, the expression of miR-21 is increased in kidney tissue and is involved in the growth of kidney cysts.

When the pharmacokinetics of SNA in mice to which disulfoCy5-anti-miR-21 (SNA) had been administered was evaluated, a strong accumulation was observed in the kidneys, and uptake into renal tubular cells was confirmed, as shown in Fig. 4.

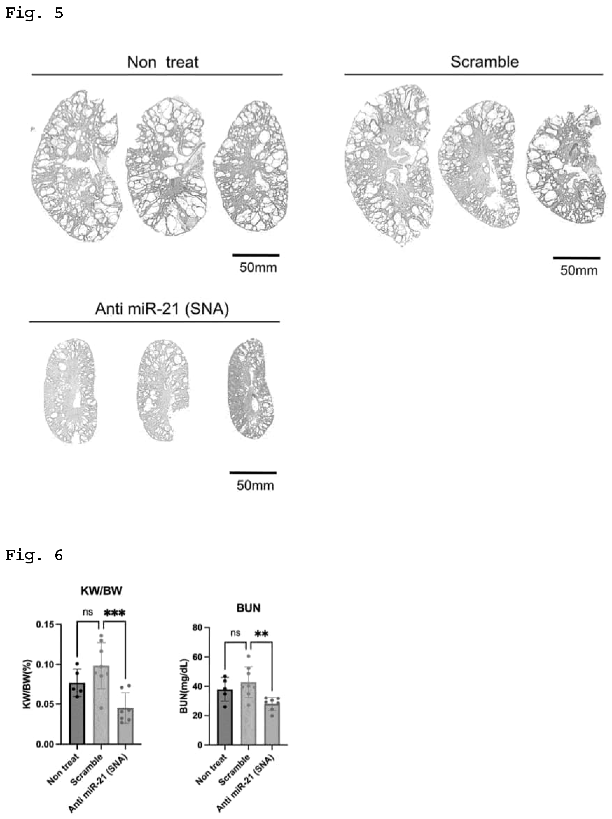

When kidney tissues of a non-treated group, a scramble administration group, and an anti-miR-21 (SNA) administration group were stained, suppression of cyst growth was observed in the SNA administration group, as shown in Fig. 5. In addition, in an anti-miR-21 (LNA) administration group, although there were large individual differences compared with the SNA administration group, some individuals also showed similar effects.

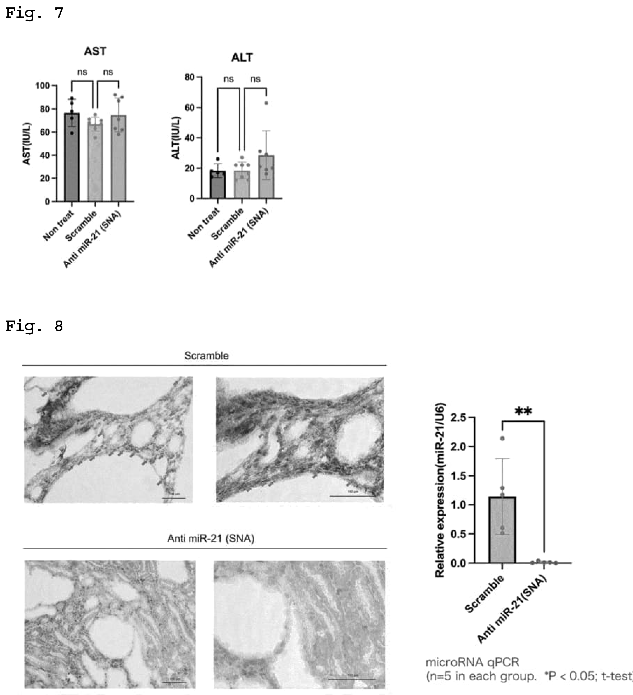

Kidney weight/body weight (KW/BW) and blood urea nitrogen (BUN) concentrations were compared between a non-treated group, a scramble administration group, and an anti-miR-21 (SNA) administration group. As shown in Fig. 6, in the SNA administration group, decreases in KW/BW and BUN concentration were confirmed, and suppression of cyst growth and improvement in kidney function were observed. On the other hand, no liver dysfunction was observed (Fig. 7). In addition, in an anti-miR-21 (LNA) administration group, although there were large individual differences compared with the SNA administration group, some individuals also showed similar effects.

The expression of miR-21 in kidney tissues of a non-treated group, a scramble administration group, and an anti-miR-21 (SNA) administration group was analyzed using miRNAscope. As shown in Fig. 8, it was confirmed that the expression of miR-21 was decreased in the SNA administered group.

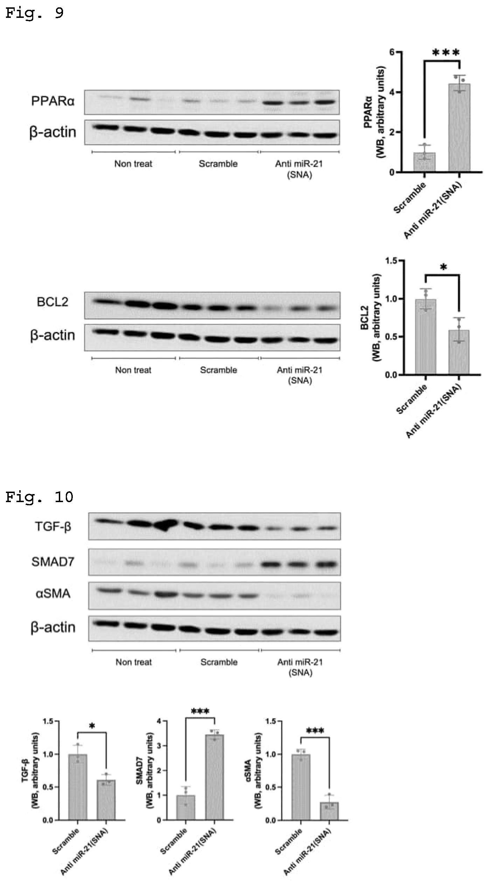

The expression of PPARα and BCL2 in kidney tissues of a non-treated group, a scramble administration group, and an anti-miR-21 (SNA) administration group was evaluated. It is known that the expression of PPARα is suppressed by miR-21, contributing to cyst enlargement through mitochondrial metabolism. Furthermore, it is known that the expression of BCL2, an anti-apoptotic protein, is increased by miR-21, contributing to cyst enlargement. As shown in Fig. 9, in the SNA administration group, promoted expression of PPARα and suppressed expression of BCL2 were confirmed. In addition, in an anti-miR-21 (LNA) administration group, although there were large individual differences compared with the SNA administration group, some individuals also showed similar effects.

The expression of TGF-β, SMAD7, and αSMA in kidney tissues of a non-treated group, a scramble administration group, and an anti-miR-21 (SNA) administration group was evaluated. It is known that the expression of SMAD7 is suppressed by miR-21, and the expression of TGF-β and αSMA is increased by miR-21, contributing to fibrosis. As shown in Fig. 10, in the SNA administration group, promoted expression of SMAD7 and suppressed expression of TGF-β and αSMA were confirmed. In addition, in an anti-miR-21 (LNA) administration group, although there were large individual differences compared with the SNA administration group, some individuals also showed similar effects.

Primary culture of renal tubular cells was performed from kidneys removed from patients with human cystic kidney disease, and three-dimensional culture was carried out. The cyst formation suppression effect of a group to which the scramble was added and a group to which anti-miR-21 (SNA) was added in the culture was evaluated. As shown in Fig. 11, cyst formation suppression was confirmed in the SNA administration group.

Serum creatinine (Cre) concentrations were compared between a scramble administration group and an S-45371 administration group. As shown in Fig. 12, the SNA (S-45371) administration group showed a decrease in Cre concentration and improvement in kidney function.

Kidney weight/body weight (KW/BW) and serum creatinine (Cre) concentrations were compared between a scramble administration group and a T-D7sU3 (PS) administration group. As shown in Fig. 13, the SNA (T-D7sU3 (PS)) administration group showed decreases in KW/BW and Cre concentration, indicating suppression of cyst growth and improvement in kidney function.

## Claims

1. A prophylactic and/or therapeutic agent for cystic kidney disease, comprising a single-stranded oligonucleotide comprising a base sequence complementary to at least a portion of a base sequence of miR-21.

2. The agent according to claim 1, wherein the single-stranded oligonucleotide comprises a modified nucleoside structural unit and/or a modified internucleoside linkage.

3. The agent according to claim 2, wherein the modified nucleoside structural unit is an acyclic nucleoside structural unit, or
the modified internucleoside linkage is a phosphorothioate linkage.

4. The agent according to claim 3, wherein the acyclic nucleoside structural unit is a structural unit represented by the following formula (1): 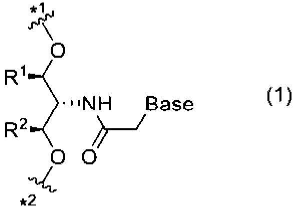 wherein R¹ and R² are the same or different, and each represents a hydrogen atom or an organic group, excluding a case in which R¹ and R² are both organic groups, and Base represents a nucleobase.

5. The agent according to claim 4, wherein in formula (1), R¹ is a hydrogen atom or a methyl group, and R² is a hydrogen atom.

6. The agent according to claim 1 or 2, wherein in the single-stranded oligonucleotide,
a base complementary to at least one uracil selected from the group consisting of positions 5, 6, 8, 14, 17, 19, and 20 of the base sequence set forth in SEQ ID NO: 1 is 2,6-diaminopurine, and/or
a base complementary to at least one adenine selected from the group consisting of positions 7, 10, and 16 of the base sequence set forth in SEQ ID NO: 1 is 2-thiouracil.

7. The agent according to claim 3, wherein
(1) the single-stranded oligonucleotide comprises a base sequence complementary to at least 18 bases of the base sequence of miR-21,
(2) a nucleoside structural unit forming the single-stranded oligonucleotide is a modified nucleoside structural unit represented by the following formula (1): 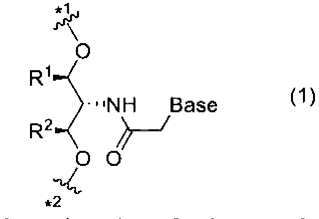 wherein R¹ and R² are the same or different, and each represents a hydrogen atom or an organic group, excluding a case in which R¹ and R² are both organic groups, and Base represents a nucleobase,
(3) in the single-stranded oligonucleotide, a base complementary to at least one uracil selected from the group consisting of positions 5, 6, 8, 14, 17, 19, and 20 of the base sequence set forth in SEQ ID NO: 1 is 2,6-diaminopurine,
(4) a base complementary to at least one adenine selected from the group consisting of positions 7, 10, and 16 of the base sequence set forth in SEQ ID NO: 1 is 2-thiouracil, and
(5) the number of phosphorothioate linkages is 20% or more based on the number of internucleoside linkages in the single-stranded oligonucleotide taken as 100%.

8. The agent according to claim 7, wherein the single-stranded oligonucleotide is
(iii-a): an oligonucleotide in which all nucleoside structural units are SNA structural units and/or L-aTNA structural units, and the base sequence of the oligonucleotide consists of the base sequence set forth in SEQ ID NO: 6, or
(iii-b): an oligonucleotide comprising a base sequence in which one or more bases are deleted, substituted, or added in the base sequence of the oligonucleotide (iii-a).

9. The agent according to claim 1 or 2, wherein the single-stranded oligonucleotide is
(i-a): an oligonucleotide consisting of the base sequence set forth in SEQ ID NO: 2,
(i-b): an oligonucleotide comprising a base sequence in which one or more bases are deleted, substituted, or added in the base sequence of the oligonucleotide consisting of the base sequence set forth in SEQ ID NO: 2,
(ii-a): an oligonucleotide in which the base sequence of the oligonucleotide is GATAAG^{m}CT, wherein ^{m}C represents 5-methylcytosine, all nucleoside structural units are LNA structural units, and all internucleoside linkages are phosphorothioate linkages, or
(ii-b): an oligonucleotide comprising a base sequence in which one or more bases are deleted, substituted, or added in the base sequence of the oligonucleotide (ii-a).
